# FASCICULE DE BREVET EUROPEEN

(11) **EP 3 390 648 B1**
(45) Date de publication et mention de la délivrance du brevet: **04.11.2020**
(21) Numéro de dépôt: 16823188.4
(22) Date de dépôt: 14.12.2016
(51) Int. Cl.: C12P 7/04, C12P 7/54, C12M 1/00

(54) **PROCÉDÉ DE SYNTHÈSE DE MOLÉCULES ORGANIQUES**
VERFAHREN ZUR SYNTHESE ORGANISCHER MOLEKÜLE
METHOD FOR SYNTHESIZING ORGANIC MOLECULES

(30) Priorité: 18.12.2015 FR 1562789
(43) Date de publication de la demande: 24.10.2018
(73) Titulaire: SUEZ Groupe, 92040 Paris la Défense Cedex (FR); Institut national de recherche pour l'agriculture, l'alimentation et l'environnement, 75007 Paris (FR); Centre National de la Recherche Scientifique, 75016 Paris (FR); Institut National Polytechnique, 31400 Toulouse (FR)
(72) Inventeur: BERGEL, Alain, 31500 Toulouse (FR); BLANCHET, Elise, 31570 Bourg-Saint-Bernard (FR); ERABLE, Benjamin, 81500 Giroussens (FR); ETCHEVERRY, Luc, 31450 Montlaur (FR); RAFRAFI, Yan, 31320 Castanet Tolosan (FR); BOUCHEZ, Théodore, 91360 Villemoisson (FR); HUYARD, Alain, 78130 Les Mureaux (FR)
(74) Mandataire: IPAZ
(86) Numéro de dépôt international: PCT/EP2016/081050
(87) Numéro de publication internationale: WO 2017/102871

(56) Documents cités:
- WO-A1-98/00558
- WO-A1-2010/068994
- WO-A1-2011/056183
- US-B2- 6 881 759
- HENSTRA ET AL: "Microbiology of synthesis gas fermentation for biofuel production", CURRENT OPINION IN BIOTECHNOLOGY, LONDON, GB, vol. 18, no. 3, 8 juin 2007 (2007-06-08), pages 200-206, XP022110181, ISSN: 0958-1669, DOI: 10.1016/J.COPBIO.2007.03.008 cité dans la demande
- MICHAEL KÖPKE ET AL: "Fermentative production of ethanol from carbon monoxide", CURRENT OPINION IN BIOTECHNOLOGY, vol. 22, no. 3, 1 juin 2011 (2011-06-01), pages 320-325, XP055104855, ISSN: 0958-1669, DOI: 10.1016/j.copbio.2011.01.005 cité dans la demande
- ZHAO T S ET AL: "A novel low-temperature methanol synthesis method from CO/H2/CO2 based on the synergistic effect between solid catalyst and homogeneous catalyst", CATALYSIS TODAY, ELSEVIER, NL, vol. 149, no. 1-2, 15 janvier 2010 (2010-01-15), pages 98-104, XP026815666, ISSN: 0920-5861 [extrait le 2009-08-18]
- Albert Kozolowski: "Données numériques sur les aciers inoxydables" In: "Técniques de l'ingenieur, traité Matériaux métalliques", December 1997 (1997-12) * Formulaire M323; page 1 - page 2 *
- ELISE BLANCHET ET AL: "Coupled iron-microbial catalysis for CO2 hydrogenation with multispecies microbial communities", CHEMICAL ENGINEERING JOURNAL, vol. 346, 1 August 2018 (2018-08-01), pages 307-316, XP055693526, AMSTERDAM, NL ISSN: 1385-8947, DOI: 10.1016/j.cej.2018.03.191

## Description

La présente invention se rapporte à un procédé de synthèse de molécules organiques à partir de sources carbonées et de dihydrogène ainsi qu'à un dispositif apte à mettre en œuvre ledit procédé. Le procédé selon l'invention peut utiliser des sources carbonées et/ou du dihydrogène issus de ressources renouvelables.

L'épuisement des ressources mondiales de combustibles fossiles et la prise de conscience croissante de l'éventuel effet anthropique sur le changement climatique conduisent à réduire de plus en plus les émissions de gaz à effet de serre pour développer une société plus durable.

Les impacts économiques, environnementaux et politiques de l'économie basée sur le pétrole ont entraîné le développement de procédés qui convertissent les produits renouvelables en carburant et en produits chimiques qui peuvent remplacer ceux dérivés du pétrole. Deux objectifs importants de ces processus de développement comprennent la compétitivité des coûts avec des processus issus du pétrole et la réduction des émissions de gaz à effet de serre.

Une approche pour la réalisation de ces objectifs est le développement de procédés de conversion de H₂ et de CO₂ en composés chimiques pouvant être stockés et transportés facilement ainsi qu'être utilisés en tant que carburant ou exploités en industrie de synthèse chimique. Ainsi, Williams R. et al. (Appl. Phys. Lett. 1978, 33, 381-383) décrivent la conversion réversible de H₂ et de CO₂ en formiate afin de pouvoir stocker de l'hydrogène tout en s'affranchissant des contraintes qui y sont liées du fait du potentiel explosif et diffusif de l'hydrogène.

De nombreuses études portent sur la mise en place de catalyse homogène ou hétérogène d'hydrogénation du CO₂ en molécules organiques d'intérêt. Ainsi, Moret S. et al. (Nat. Commun. 2014, 5, 4017-4023) décrivent une catalyse d'hydrogénation du CO₂ en formiate tandis que H. Li et al. (Science 2012, 335, 1596-1596) décrivent une catalyse d'hydrogénation du CO₂ en isobutanol et 3-méthyl-1-butanol à l'aide de *Ralstonia eutropha* H16, un microorganisme chimiolithoautotrophe génétiquement modifié.

Cette catalyse microbienne pour la production de molécules organiques d'intérêt présente un grand avantage puisque les microorganismes utilisés offrent une grande potentialité de conversion de H₂ et de CO₂ et qu'en plus les microorganismes sont les propres catalyseurs de ces réactions catalytiques.

Jusqu'à aujourd'hui, les catalyses microbiennes sont essentiellement mises en œuvre avec des cultures pures et l'utilisation de consortia microbiens est rare.

La gazéification de la biomasse conduit à la production de gaz de synthèse (syngas) qui est un intermédiaire clé dans la synthèse de carburants synthétiques. Ce gaz est composé essentiellement de monoxyde de carbone et de molécules d'hydrogène qui servent de briques (building blocks) dans des procédés comme la synthèse Fischer-Tropsch pour former des alcanes linéaires. Henstra A.M. et al. (Curr Opin Biotechnol, 2007, 18:1-7) est une revue sur la fermentation de ce gaz de synthèse par des microorganismes qui conduit à la production de carburant et de divers produits chimiques comme l'acétate, l'éthanol, le butanol, le butyrate et l'hydrogène pur.

Une autre approche consiste à développer les procédés de bioraffinage utilisant des microorganismes pour convertir les sources de matières premières renouvelables comme la biomasse cellulosique ou des déchets en produits qui sont traditionnellement dérivés du pétrole ou qui peuvent remplacer les produits dérivés du pétrole. En raison d'une concurrence croissante entre le bioraffinage et la consommation alimentaire de céréales telles que le maïs et le sucre, il est clair que la bioproduction de carburants et de produits chimiques nécessitera l'utilisation d'une large gamme de matières premières renouvelables alternatives.

Ainsi le brevet EP0909328 décrit un procédé de production d'un acide organique, d'alcool ou d'hydrogène par fermentation d'un gaz d'échappement issu d'un processus industriel, ledit gaz d'échappement contenant du CO et de l'eau ou du CO₂ et de l'hydrogène ; le procédé est mis en œuvre en présence d'un milieu nutritif aqueux dans un bioréacteur avec des bactéries anaérobies capables de convertir le gaz d'échappement en l'un des produits attendus.

Aussi, la demande WO2015172972 décrit un procédé de production de composés organiques comprenant la mise en contact d'une culture mixte de microorganismes, acétogènes et non-acétogènes, avec de l'oxygène et une source de carbone. Les microorganismes acétogènes convertissent la source de carbone en acétate et/ou en éthanol alors que les microorganismes non-acétogènes métabolisent l'acétate et/ou l'éthanol en composés organiques.

Pour être applicables à l'échelle industrielle, ces procédés de synthèse de molécules organiques à partir de source de carbone renouvelables devront être optimisés tant du point de vue de la mise en œuvre que de la cinétique et du rendement.

Une autre voie de conversion de composés renouvelables en molécules organiques d'intérêt est l'électrosynthèse microbienne. On entend par « électrosynthèse microbienne » la production de composés organiques par la réduction d'au moins une source de carbone grâce à l'action de microorganismes actifs électrochimiquement présents dans un compartiment cathodique, couplé à un compartiment anodique où est réalisée l'oxydation de composés organiques oxydables (par exemple déchets organiques), également sous l'action de microorganismes actifs électrochimiquement. De tels procédés sont bien connus de l'homme du métier et sont notamment décrits dans la demande internationale WO2010068994.

Lorsque les microorganismes sont actifs électrochimiquement et qu'ils interagissent avec une anode (en transférant des électrons à l'anode), une telle électrode est appelée anode biologique, bioanode, ou anode microbienne, et les microorganismes sont qualifiés d'anodophiles. En revanche, lorsque les microorganismes sont actifs électrochimiquement et qu'ils interagissent avec une cathode (en acceptant des électrons de la cathode), une telle électrode est désignée en tant que cathode biologique, biocathode, ou cathode microbienne, et les microorganismes sont qualifiés de cathodophiles.

Ainsi la demande internationale WO2010068994 décrit un procédé de production de composés chimiques utilisant un système bioélectrochimique constitué d'une anode et d'une cathode séparées l'une de l'autre par une membrane. Ce système électrochimique fournit des équivalents réducteurs et du CO₂, par le biais des réactions d'oxydation, au niveau de l'anode, et de réduction, au niveau des cathodes, à une culture de micro-organismes permettant la production de composés chimiques complexes.

La demande internationale WO2011087821 concerne des systèmes et des procédés de génération de composés organiques en utilisant du CO₂ en tant que source de carbone et un courant électrique en tant que source d'énergie. Le système selon la demande WO2011087821 comprend une cellule de réaction constituée d'une anode et d'une cathode séparées l'une de l'autre par une membrane perméable.

La demande internationale WO2014043690 décrit des méthodes d'électrosynthèse microbienne permettant la production de matières plastiques biodégradables et renouvelables en utilisant de l'électricité et du CO₂.

Enfin la demande internationale WO2009131452 a trait à un dispositif comprenant une bioanode, contenant un substrat pouvant être électrochimiquement oxydé et d'autres composés facultatifs, et une biocathode, contenant un substrat pouvant être électrochimiquement réduit et d'autres composés facultatifs. La bioanode et la biocathode sont électriquement connectées et présentent à leur surface un catalyseur destiné à la catalyse de la conversion électrochimique du substrat.

L'électrosynthèse microbienne requiert souvent la séparation du milieu réactionnel en deux compartiments anodiques et cathodiques au moyen d'un séparateur, notamment afin d'améliorer la sélectivité et le rendement, et aussi de répondre aux exigences réglementaires en matière sanitaire et/ou alimentaire qui pourraient exiger que les molécules organiques produites à la cathode soient très strictement isolées des déchets organiques oxydés à la cathode.

Ce séparateur pose des problèmes technologiques car il doit séparer les solutions dans lesquelles baignent la cathode et l'anode tout en assurant un transfert des ions, le plus rapide possible, entre les deux compartiments.

Il y a donc un besoin de procédés permettant de réaliser des synthèses à grande échelle de molécules organiques par conversion de composés renouvelables (par exemple CO₂ capté et dihydrogène produit par des procédés de traitement de déchets ou à partir d'énergies vertes) en molécules organiques d'intérêt. Notamment, il existe un besoin de développer de tels procédés permettant de produire des molécules organiques avec une cinétique et un rendement amélioré. Avantageusement, ce procédé devra répondre aux exigences réglementaires en matière sanitaire et/ou alimentaire pour les molécules produites.

Or les inventeurs ont découvert de manière surprenante qu'un procédé catalytique comprenant la mise en contact de microorganismes avec un catalyseur solide, en présence d'un courant de dihydrogène, pourrait permettre d'augmenter significativement la cinétique et le rendement de la synthèse de molécules organiques à partir d'une source de carbone par rapport aux procédés sans catalyseur solide dans lesquels les microorganismes sont les seuls catalyseurs.

La présente invention a donc pour objet de mettre à disposition un procédé de synthèse de molécules organiques à partir d'une source de carbone et de dihydrogène, avec une cinétique et un rendement amélioré par rapport aux procédés de l'art antérieur. La source de carbone et le courant de dihydrogène peuvent être issus de productions à partir de ressources renouvelables, sans toutefois que se posent des problèmes de contact entre les molécules organiques produites et des ressources renouvelables de type déchets organiques.

Le procédé de synthèse de molécules organiques selon l'invention comprend la mise en contact, dans un milieu liquide aqueux, en conditions anaérobies, d'au moins une source de microorganismes anaérobies capables de catalyser la réduction d'une molécule carbonée par le dihydrogène, avec au moins un courant d'hydrogène, en présence d'au moins une source de carbone et d'au moins un catalyseur solide.

Conformément à la présente invention, on entend par « microorganismes capables de catalyser la réduction d'une molécule carbonée par le dihydrogène » toutes cellules chimioorganotrophes ou chimiolithotrophes utilisant la matière organique ou minérale préexistante à la fois comme source d'énergie et de carbone et de pouvoir réducteur, ou en d'autres termes capables de transférer des électrons au substrat carboné afin de faciliter leur réduction. Ce sont par exemple les microorganismes cathodophiles décrits dans les procédés connus d'électrosynthèse microbienne.

Sans vouloir être lié par une quelconque théorie, le catalyseur solide selon l'invention pourrait permettre la fixation à sa surface des molécules de dihydrogène, des microorganismes et éventuellement du substrat carboné à hydrogéner facilitant ainsi la réaction globale.

Le procédé de l'invention peut être mis en œuvre simplement dans un contacteur gaz-liquide ou gaz-solide-liquide, technologie de base des industries de procédés, et en particulier des industries pétrolières (par exemple, hydrogénations ou oxygénations catalytiques), et qui est une technologie parfaitement maîtrisée à très grande échelle.

Le procédé de synthèse de molécules organiques selon l'invention ne nécessite pas impérativement la coexistence de plusieurs types de microorganismes à mécanisme d'action différents, par exemple acétogènes et non acétogènes. Les molécules sont directement synthétisées à partir de l'alimentation par un courant de dihydrogène et de la source de carbone, en présence du système catalytique selon l'invention.

Conformément à l'invention, le procédé peut comprendre en outre une étape de récupération des molécules organiques synthétisées pour leur utilisation ultérieure.

Dans le cadre de l'invention, on entend par « milieu liquide aqueux » tout milieu de culture approprié pour les microorganismes ensemencés dans le contacteur et comprend tous les éléments nutritifs nécessaires à leur bon développement et favorisant ainsi leur métabolisme. Ces milieux sont bien connus de l'homme du métier qui saura choisir le milieu en fonction des microorganismes utilisés et des molécules organiques souhaitées.

Les sources de microorganismes du procédé selon l'invention peuvent par exemple comprendre les microorganismes cathodophiles décrits dans les procédés connus d'électrosynthèse microbienne.

Ainsi, la demande WO2010/068994 divulgue l'utilisation de *Cupriviavidus necator* (*Alcaligenes eutrophus* ou *Ralstonia eutropha*) pour la production de biopolymères de type poly-beta-hydroxybutyrate (PHB). Liou et al., (Int. Syst. Evol. Microbiol., 2005, 55, 2085-2091) divulguent l'utilisation de *Clostridium carboxidivorans sp nov* pour la production d'acétate, d'éthanol, de butyrate et de butanol à partir de dioxide de carbone par électrosynthèse microbienne. La demande WO2011/087821 divulgue l'utilisation de bactéries du genre *Geobacter, sporomusa, Clostridium* et *Moorella* comme microorganismes cathodophiles pour la production de molécules organiques par électrosynthèse microbienne.

Les procédés selon l'invention peuvent aussi être mis en oeuvre avec des microorganismes capables de se développer en milieu anaérobie à hautes concentrations salines (par exemple des concentrations en NaCI de l'ordre de quelques grammes à dizaines de grammes par litre), par exemple du genre *Geobacter, Vibrio, Bacillus, Halothermothrix, Halanaerobium, Halocella.* Les microorganismes du genre *Halobacterium, Selenihalanaerobacter, Methanomicrobiales* sont également adaptés et peuvent se développer dans des milieux de salinité encore plus élevée.

Le courant de dihydrogène gazeux peut être soit un courant de dihydrogène seul, soit un mélange de dihydrogène avec un autre gaz comme par exemple un mélange CO₂/H₂, soit un mélange CO/H₂. Ces mélanges peuvent contenir d'autres gaz nécessaires au développement du microorganisme utilisé ou un gaz neutre destiné à réguler les pressions partielles comme N₂ par exemple.
Avantageusement, le CO₂ et le CO peuvent servir de source de carbone à l'au moins une source de microorganismes.

Dans le cadre de l'invention, le CO₂, ou le CO, et le H₂ sont présents dans des proportions proches du rapport stœchiométrique de la molécule synthétisée souhaitée. L'homme du métier saura choisir ces proportions au regard de ses connaissances générales.

Dans un mode de réalisation avantageux de l'invention, tout ou partie du courant de dihydrogène provient d'une électrolyse microbienne comprenant l'oxydation de déchets organiques. De tels procédés sont par exemple décrits dans la demande WO2005005981. Ils mettent en œuvre, dans un réacteur électrochimique comprenant une anode, une cathode, et un milieu aqueux contenant des bactéries électroactives anodophiles, capables de bio-oxyder un substrat organique, et de transférer des électrons à l'anode. Sous l'effet de la différence de potentiel appliquée entre l'anode et la cathode, les électrons produits par l'oxydation de déchets organiques bio-oxydables introduits dans le milieu aqueux sont transférés à la cathode, où se produit un dégagement de dihydrogène.

En particulier, les déchets organiques peuvent être des eaux usées industrielles, agricoles et domestiques ou des déchets d'origine domestique, industrielle, végétale ou animale.

Par rapport aux procédés d'électrosynthèse microbienne connus, où la séparation des déchets organiques oxydés et des molécules d'intérêt produites est délicate, ce mode de réalisation particulier de l'invention permet de répondre aux exigences d'isolation stricte entre les molécules organiques d'intérêt produites et les déchets organiques.

En effet, on peut produire de l'hydrogène à partir de déchets organiques dans une cellule d'électrolyse microbienne, récupérer le flux de dihydrogène ainsi produit et l'envoyer alimenter un réacteur de synthèse de molécules organiques distant séparé.

Selon un mode de réalisation avantageux de l'invention, tout ou partie du courant de dihydrogène provient d'une digestion anaérobie de déchets organiques. Ce mode de réalisation permet également de résoudre le problème de la séparation des déchets organiques et des molécules organiques produites, puisque la digestion anaérobie peut être distante et séparée de la production de molécules organiques.

Selon un mode de réalisation avantageux de l'invention, tout ou partie du courant de dihydrogène provient de l'électrolyse de l'eau. Selon un mode particulièrement avantageux, cette électrolyse de l'eau est réalisée avec une production d'électricité par des sources d'énergies renouvelables, telles que par exemple l'énergie éolienne ou solaire, de manière par exemple à absorber les excédents ponctuels de production.

Selon un mode de réalisation avantageux de l'invention, tout ou partie du courant de dihydrogène comprend un mélange de dihydrogène produit par au moins deux des méthodes de production de dihydrogène décrites ci-dessus.

Conformément à l'invention, la au moins une source de carbone est
a. soit sous forme gazeuse et choisie parmi le monoxyde de carbone et le dioxyde de carbone,
b. soit en solution et choisie parmi des solutions comprenant des carbonates, des carboxylates en C₁-C₈, des sucres, du lactate et les mélanges de ceux-ci.

Lorsque la au moins une source de carbone est sous forme gazeuse et choisie parmi le monoxyde de carbone et le dioxyde de carbone, les procédés selon l'invention contribuent à résoudre les problèmes de captation de ces gaz dans l'environnement et utilisent une source de carbone renouvelable.

Dans la présente invention on peut citer comme exemple de carboxylates en C₁-C₈ : le méthanoate (ou formiate), l'acétate, le butyrate, le propionate, le pentanoate, l'hexanoate (ou caproate), l'heptanoate et l'octanoate.

Dans la présente invention on peut citer comme exemple de carbonates : les carbonates de calcium, les carbonates de magnésium, les carbonates de potassium, les carbonates de sodium, les carbonates de fer, les carbonates de manganèse et tout autre carbonate approprié au regard des connaissances de l'homme du métier.

Conformément à l'invention, on entend par sucre ou glucide, notamment le glucose, le lactose et le xylose, ou tout autre sucre ou glucide approprié au regard des connaissances de l'homme du métier.

On entend par « mélanges de ceux-ci » des mélanges d'au moins deux ou plus de ces différentes sources de carbone.

L'au moins un catalyseur solide selon l'invention permet la fixation à sa surface des molécules de dihydrogène et des microorganismes et éventuellement du substrat carboné à hydrogéner. Conformément à l'invention, il peut être choisi parmi les matériaux conducteurs électriques ou semi-conducteurs.

Dans le cadre de l'invention, on entend par « conducteur » tout matériau capable de laisser passer un courant électrique et/ou de transférer des électrons entre espèces chimiques ou biologiques adsorbées à sa surface et on entend par « semi-conducteur » tout matériau pouvant soit se comporter comme un isolant électrique soit comme un conducteur, en fonction des conditions dans lesquelles il est placé. Des exemples de matériaux semi-conducteurs sont par exemple le silicium, dopé ou non.

Avantageusement, l'au moins un catalyseur solide est choisi parmi un catalyseur d'hydrogénation ou un catalyseur comprenant des matériaux carbonés, ou leur mélange.

Dans la présente invention, on entend par « matériau carboné » tout matériau composé essentiellement d'atomes de carbone. Lesdits matériaux carbonés peuvent être choisis parmi le carbone, le graphite, le graphène, les nanotubes de carbone, le diamant ou toute autre forme de matériau carboné connu de l'homme du métier.

Le catalyseur solide selon l'invention est préférentiellement choisi parmi les matériaux métalliques, c'est-à-dire constitués d'un métal ou d'un alliage ou d'un mélange de métaux (catalyseur massif) y compris leurs oxydes, ou bien comprenant un ou plusieurs métaux fixés sur un support (catalyseur supporté).

Les métaux des catalyseurs métalliques solides selon l'invention sont notamment choisis parmi le fer, le ruthénium, le cobalt, le nickel, le chrome, le platine, le palladium, le rhodium, ou un mélange de ceux-ci, y compris un mélange de leurs oxydes. Le fer, le cobalt, le nickel et le ruthénium seront préférés, et tout particulièrement le fer.

Le cuivre, le zinc, le chrome ou l'aluminium peuvent également être choisis, seuls ou en mélange, ou en mélange avec les métaux précédemment cités, par exemple cobalt-cuivre, ou zinc-aluminium.

Le catalyseur solide peut être :
- de l'acier doux, ou de l'acier au carbone, lorsque le fer est mélangé avec des matières carbonées, ou
- de l'acier inoxydable, lorsque le fer est mélangé avec du nickel ou du chrome, y compris leurs oxydes.

Les mélanges utilisés comme catalyseur selon l'invention peuvent également être tout autre mélange apte à être utilisé comme catalyseur solide approprié au regard des connaissances de l'homme du métier.

Les molécules organiques synthétisées par le procédé selon l'invention sont fonction de la composition dudit catalyseur solide, de la source de carbone, ainsi que de la au moins une source de microorganismes. Des exemples sont donnés dans le tableau 1 qui suit :

**TABLEAU 1**

| Références | Microorganismes | Substrats | Molécules produites |
|---|---|---|---|
| D. D. Woods, Biochem. J. 1936, 30, 515-527 | Echerischia Coli | H₂/CO₂ | formiate |
| US 2012/0288898 Losley et Nevin | *Sporomusa ovata* (DSM 2662) | H₂/CO₂ (80/20) | acétate |
| | *Sporomusa sphhaeroides* (DSM 2875) | | |
| | *Clostridium Ijungdahlii* (DSM 13528) | | |
| | *Clostridium aceticum* (DSM 1496) | | |
| | *Moorella thermoacetica* (DSM 21394) | | |
| | *Acetobacterium woodii* (DSM 1030) | | |
| F. Barbirato et al (1998) Industrial crops and products, 7, 2-3, pp 281 - 289 | *Klebsiella pneumniae Citrobacter freundii Enterobacter agglomerans Clostridium butyricum* | glycérol | 1,3 propanediol |
| | | vinasses de distillation | |
| | *Clostridium butyricum* | | |
| US 2014/0322772 Angenent et Agler | Microorganismes ou mélange de microorganismes | éthanol + Acide Gras Volatiles | caproate, caprilate |
| WO 2010/068994 Rozendal et Rabaey | *Cupriviadus necator* (*Alcaligenes eutrophus* ou *Ralstonia eutropha*) | H₂/CO₂ | Biopolymères, poly-beta-hydroxybutyrate (PHB) |
| Liou et al (2005) Int. Syst. Evol. Microbiol., 55, pp 2085 - 2091 | *Clostridium carboxidivorans* | H₂/CO₂ | acétate, éthanol, butyrate, butanol |
| WO 2011/087821 Lovley et Nevin | *Geobacter* | CO2 | Composés chimiques avec au moins 2C |
| | *Sporomusa* | | |
| | *Clostridium* | | |
| | *Moorella* | | |

Selon un aspect de l'invention, ledit catalyseur solide se présente sous forme d'une grille, d'une plaque ou d'une structure tridimensionnelle ou poreuse. Ladite structure tridimensionnelle ou poreuse pouvant être constituée de billes, de granules, de poudre ou d'une superposition de grilles, de plaques ajourées ou déployées ou toute autre structure tridimensionnelle ou poreuse connue de l'homme du métier permettant d'augmenter la surface de contact avec les microorganismes, du dihydrogène et de la au moins une source de carbone.

Selon un mode de réalisation avantageux de l'invention, le matériau conducteur poreux a une porosité supérieure au micromètre, de façon optimale de 50 à 5000 µm, avantageusement de 200 à 2000 µm, et de manière encore plus avantageuse de 1000 à 2000 µm pour permettre l'accès des microorganismes à un maximum de surface, et éventuellement de s'y fixer, en tout ou partie, pour s'y développer.

Selon un autre mode de réalisation particulier de l'invention, ledit catalyseur solide est une membrane métallique et ledit catalyseur est le diffuseur d'au moins un courant gazeux.

Selon un aspect particulier de l'invention, le catalyseur d'hydrogénation solide peut servir de support à l'au moins une source de microorganismes, auquel cas il entraîne la formation d'un biofilm.

Dans la présente invention, on entend par « biofilm » une communauté multicellulaire plus ou moins complexe, pouvant être symbiotique ou non, de microorganismes, pouvant adhérer totalement ou partiellement à la surface dudit catalyseur d'hydrogénation et adhérant entre eux.

Selon la présente invention, la source de microorganismes est choisie dans le groupe comprenant les cultures pures, les co-cultures, les inocula environnementaux et des consortia microbiens complexes.

Au sens de l'invention, l'au moins une source de microorganismes sont des microorganismes anaérobies, ceux-ci pouvant être des microorganismes anaérobies stricts ou des microorganismes anaérobies facultatifs.

Dans la présente invention, on entend par « inocula environnementaux » tout échantillon comprenant des germes vivants que l'on introduit dans un milieu favorable en vue de la multiplication ou de l'identification desdits germes.

Avantageusement, les inocula environnementaux selon l'invention peuvent être : des déchets d'origine domestique, industrielle, végétale ou animale ; du limon ; du lisier ; de la terre ; des eaux usées telles que les eaux usées domestiques, industrielles ou de toute autre origine, par exemple des inocula de marais salants.

Selon un mode de réalisation avantageux de l'invention, lesdits microorganismes peuvent:
- soit provenir directement de la source de carbone telle qu'on peut la trouver dans son environnement, ou
- soit être ajoutée après stérilisation de la source de carbone.

Selon un mode de réalisation avantageux de l'invention, la source de microorganismes est ajoutée après la stérilisation de la au moins source de carbone permettant ainsi de s'assurer de la sélectivité des molécules organiques produites.

Le procédé de synthèse selon l'invention permet de produire des molécules organiques complexes. Parmi ces molécules on peut citer les hydrocarbures, les alcools, des acides carboxyliques, par exemple des carboxylates tels que le formiate, l'acétate, le caproate, des acides dicarboxyliques tels que l'acide itaconique, des acides carboxyliques hydroxylés, par exemple alpha hydroxylés, tels que glycolate, lactate, les biopolymères, par exemple les polyhydroxyalcanoates (PHA), par exemple le poly-beta-hydroxybutyrate (PHB).

Les molécules organiques produites par les procédés selon l'invention comprennent généralement entre 1 et 12 atomes de carbones, en particulier entre 2 et 10 atomes de carbone, en particulier entre 4 et 8 atomes de carbone.

Les molécules organiques produites peuvent comporter des chaines carbonées courtes, et comporter entre 1 et 3, ou entre 2 et 6 atomes de carbone.

Les molécules organiques produites peuvent également comporter des chaines carbonées suffisamment longues pour leur conférer des propriétés lubrifiantes ou tensio-actives.

Avantageusement lorsque lesdites molécules organiques sont des alcools, ces derniers sont de préférence l'éthanol, le méthanol, le butanol, l'isobutanol, le 1,3 propanediol et le 1,4 butanediol.

Avantageusement, lorsque lesdites molécules organiques sont des acides carboxyliques, ces derniers comportent généralement entre 1 et 12 atomes de carbone, ou encore entre 2 et 10 atomes de carbone, ou entre 4 et 8 atomes de carbone, comme par exemple le méthanoate (ou formiate), l'acétate, le butyrate, le succinate, le propionate, le pentanoate, l'hexanoate (ou caproate), l'heptanoate, l'octanoate, le nonanoate, le décanoate et le 2,5 furanedicarboxylique.

Dans un mode de réalisation particulier, le procédé selon l'invention permet de produire des hydrocarbures par le biais d'une réaction de Fischer-Tropsch microbien, c'est-à-dire par fermentation de H₂ + CO (Henstra AM, et al. (2007), Curr Opin Biotechnol 18:1-7*;* Köpke M. et al. (2011), Current Opinion in Biotechnol, 22, 320-325).

La réaction Fischer-Tropsch fait intervenir la catalyse de monoxyde de carbone et d'hydrogène en vue de les convertir en hydrocarbures. Dans ce procédé chimique, les catalyseurs d'hydrogénation solides les plus courants sont le fer, le cobalt ou le ruthénium, ainsi que le nickel. L'intérêt de la conversion est de produire du pétrole brut de synthèse à partir de charbon ou de gaz.
La réaction Ficher-Tropsch est la suivante :

(2n+1)H₂ + nCO -> CₙH₂ₙ₊₂ + nH₂O

Dans les procédés selon l'invention, n est un nombre entier généralement compris entre 1 et 20, plus particulièrement entre 2 et 12, plus particulièrement entre 4 et 8.

Un autre aspect de l'invention concerne un dispositif apte à mettre en œuvre le procédé selon l'invention, ledit dispositif comprenant :
- des moyens d'arrivée d'au moins un courant gazeux comprenant du dihydrogène,
- au moins une source de carbone,
- au moins une source de microorganismes,
- au moins un moyen de mise en contact dudit au moins un courant gazeux et desdits microorganismes, et
- un catalyseur solide.

De façon avantageuse, le dispositif selon l'invention met en contact les gaz et la solution à l'aide d'un disperseur, d'un contacteur membranaire, ou de tout autre moyen connu de l'homme du métier assurant la vitesse optimale du gaz vers la solution. On entend par « vitesse de transfert optimale » la vitesse de transfert gazeux la plus proche possible de la vitesse de consommation du gaz par la réaction microbienne.

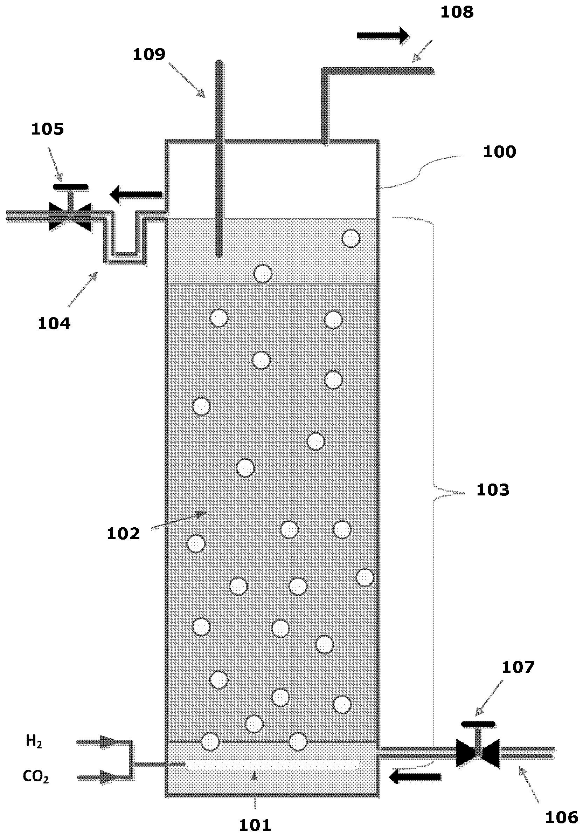

Les figures 1 à 4 et les exemples ci-après illustrent la présente invention.

La figure 1 montre un schéma expérimental d'un mode de réalisation du dispositif mettant en œuvre le procédé selon l'invention. Le dispositif est un contacteur gaz-liquide-solide fermé en atmosphère anaérobie et comprend : un récipient 100 présentant une entrée d'hydrogène gazeux (H₂) et de CO₂, un diffuseur de gaz 101 permettant de diffuser le mélange gazeux dans le récipient 100, et un catalyseur solide sous forme d'une grille 102 immergé dans le milieu de culture 103 permettant la synthèse microbienne à partir de la au moins une source de carbone entrant dans le récipient 100 par le conduit 106 régulé par une valve d'entrée 107. Une fois les molécules organiques produites, elles sortent par un conduit 104 régulé par une valve 105 afin de les récupérer. Dans la partie haute du récipient 100, un conduit 108 évacuant le gaz diffusé par le diffuseur 101 et des moyens de mesurer le pH et la teneur en hydrogène 109 sont présents.

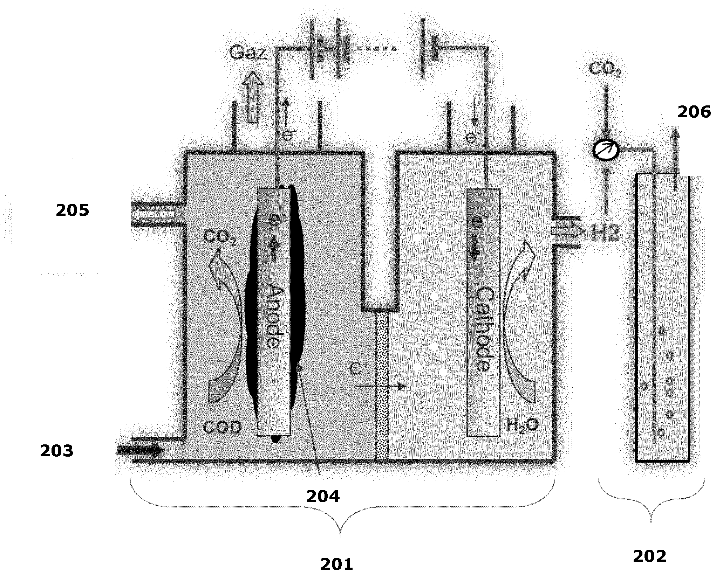

La figure 2 montre un schéma expérimental d'un mode de réalisation particulier du dispositif mettant en œuvre le procédé selon l'invention. Le dispositif comprend un compartiment d'électrolyse microbienne 201 et un contacteur gaz-liquide-solide 202. Ledit compartiment 201 comprend, au niveau de l'anode, une entrée de déchets organiques par le conduit 203 servant de substrat aux microorganismes anodophiles 204 présents sous forme de biofilm puis les effluents traités sortent par le conduit 205 ; au niveau de la cathode, le flux d'électrons (e⁻) entrant permet l'électrolyse de l'eau qui fournit au contacteur gaz-liquide-solide 202 le dihydrogène nécessaire à la réalisation du procédé selon l'invention. Les molécules organiques produites sont récupérées à la sortie 206 du contacteur gaz-liquide-solide 202.

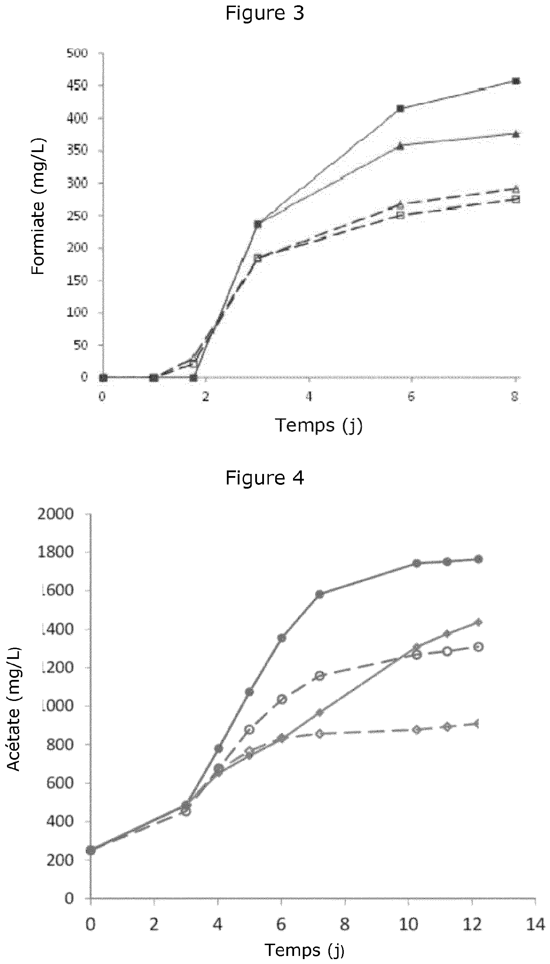

La figure 3 représente la production de formiate pendant 8 jours dans des contacteurs inoculés par des marais salants et alimentés en continu par N₂/CO₂/H₂ (40/10/50 en volume) conformément à l'exemple 1. Lesdits contacteurs inoculés sont mis soit en présence de catalyseur d'hydrogénation solide sous forme de grille avec sulfate (▲) ou sans sulfate (■), soit en l'absence de catalyseur d'hydrogénation avec sulfate (Δ) ou sans sulfate (□).

La figure 4 représente la production d'acétate pendant 12 jours dans des contacteurs inoculés par des boues de Station d'Epuration des eaux usées (STEP) et alimentés en continu par N₂/CO₂/H₂ (40/10/50 en volume) conformément à l'exemple 2. Lesdits contacteurs inoculés sont mis soit en présence de catalyseur d'hydrogénation sous forme de grille avec inhibiteur de méthanogènes (●) ou sans inhibiteur de méthanogènes (◆), soit en l'absence de catalyseur d'hydrogénation avec inhibiteur de méthanogènes (○) ou sans inhibiteur de méthanogènes (◇).

### Exemple 1 : Synthèse de formiate et d'acétate à partir de CO₂ et H₂ catalysée par un inoculum de marais salant et par de l'acier inoxydable.

Quatre contacteurs sont disposés suivant deux lignes parallèles qui comportent chacune deux contacteurs branchés en série (le mélange gazeux issu du premier est introduit dans le second). Chaque ligne est alimentée par un mélange de gaz N₂/CO₂/H₂ (40/10/50 en volume) à un débit continu de 6 mL.min⁻¹. Pour chaque ligne le premier contacteur contient un catalyseur d'hydrogénation solide sous forme de grille en acier inoxydable 316 L de 98 cm² de surface développée totale, le second ne contient pas de catalyseur d'hydrogénation.

Chaque contacteur contient 122 mL du milieu de culture composé de KCl 0,1 g/L, NH₄Cl 1,5 g/L, NaHCO₃ 2,5 g/L, NaH₂PO₄ 0,6 g/L, CaCl₂ 0,01 g/L, MgCl₂ 0,1 g/L, NaCI 45 g/L, mélange de vitamines (ATCC), 10 ml/L, trace minérales (ATCC) 10 ml/L. Chaque contacteur est inoculé à 10% (v/v) par des sédiments de marais salant fraichement récoltés dans les salines de Gruissan. Du sulfate d'ammonium à 1 mM a été ajouté dans les deux contacteurs de la seconde ligne pour tenter de favoriser le développement des bactéries sulfato-réductrices.

L'ensemble des contacteurs est placé dans un bain marie dont la température est régulée à 30°C. Des prélèvements sont effectués quotidiennement et analysés par HPLC.

La formation de formiate est observée (Figure 3) avec, au bout de huit jours, des concentrations maximales de 415 ± 40 mg.L⁻¹ en présence d'une grille et 275 ± 5 mg.L⁻¹ en l'absence d'un catalyseur d'hydrogénation sous forme de grille. L'ajout de sulfate n'a pas eu d'effet. En revanche le catalyseur augmente la concentration finale en formiate de plus de 50% comparativement à celle obtenue en l'absence de grille.

La présence de catalyseur d'hydrogénation sous forme de grille d'acier (courbes continues) a un effet significatif sur la production par rapport aux contrôles réalisés sans catalyseur d'hydrogénation (tirets). L'ajout de sulfate (noté SO₄) n'a pas d'effet significatif.

### Exemple 2 : Synthèse d'acétate à partir de CO₂ et H₂ catalysée par un inoculum de boue de station d'épuration et par de l'acier inoxydable.

Le dispositif expérimental est similaire au précédent avec un débit de gaz fixé à 18 mL.min⁻¹ et des grilles de surface totale 300 cm² dans chaque contacteur concerné. Elles sont préalablement nettoyées dans un mélange éthanol:acétone (50:50) puis décapées dans un mélange fluoro-nitrique. Chaque contacteur est ensemencé avec des boues de station d'épuration (STEP) à un taux de 10% (v/v) dans le milieu de culture qui se compose de : KCI 0,1 g/L, NH₄Cl 1,5 g/L, NaHCO₃ 2,5 g/L, NaH₂PO₄ 0,6 g/L, vitamines (ATCC) 10 mL/L, trace minérale (ATCC) 10 mL/L.

Enfin, pour limiter la consommation de l'acétate produit par les bactéries méthanogènes qui pourraient être présentes dans le consortium (selon la réaction CH₃COOH → CH₄ + CO₂) du 2-bromoethanesulfonate (BES) inhibiteur spécifique des méthanogènes, est ajouté à la concentration de 500 µM dans les deux contacteurs de la seconde ligne (notés MB5 et 6).

Les contacteurs de la première ligne (notés MB3 et 4) sont inversés, c'est-à-dire que le mélange gazeux passe d'abord dans le contacteur de contrôle (MB) sans catalyseur d'hydrogénation (◇, ○) avant le contacteur muni de catalyseur d'hydrogénation sous forme de grille (◆, ●), afin d'évaluer un éventuel effet de l'ordre des contacteurs.

On note une production d'acétate dans tous les contacteurs (Figure 4). La présence de l'inhibiteur de méthanogènes (BES) a bien inhibé les méthanogènes, ce qui explique les concentrations d'acétate plus élevées sur la ligne MB5-6 par rapport à celles de la ligne MB 3-4. Les concentrations obtenues en 10 jours sont de 1250 mg.L⁻¹ pour le contrôle et 1730 mg.L⁻¹ en présence de catalyseur d'hydrogénation sous forme de grille, soit une amélioration de 38 % due à la présence du catalyseur.
La vitesse de production maximale est de 295 mg.L⁻¹.j⁻¹ et est stable sur 3 jours (entre les jours 3 et 6) en présence de grilles, alors qu'elle est de 210 mg.L⁻¹.j⁻¹ et n'est maintenue que 2 jours pour le contrôle. Le même effet significatif de catalyseur d'hydrogénation sous forme de grille est observé en l'absence de l'inhibiteur de méthanogènes, avec des concentrations maximales moins importantes.

## Revendications

1. Procédé de synthèse de molécules organiques comprenant la mise en contact, dans un milieu liquide aqueux en conditions anaérobies, d'au moins une source de microorganismes anaérobies, capables de catalyser la réduction de molécules carbonées par le dihydrogène, avec au moins un courant de dihydrogène, en présence d'au moins une source de carbone et d'au moins un catalyseur solide **caractérisé en ce que** le catalyseur solide est un catalyseur métallique massif ou supporté.

2. Procédé selon la revendication 1 **caractérisé en ce que** tout ou partie du courant de dihydrogène provient d'une électrolyse microbienne comprenant l'oxydation de déchets organiques, et/ou d'une digestion anaérobie de déchets organiques, et/ou de l'électrolyse de l'eau.

3. Procédé selon la revendication 1 ou la revendication 2, **caractérisé en ce que** la au moins une source de carbone est
a. soit sous forme gazeuse et choisie parmi le monoxyde de carbone et le dioxyde de carbone,
b. soit en solution et choisie parmi des solutions comprenant du carbonate, des carboxylates en C₁-C₈, des sucres, du lactate, seuls ou en mélange.

4. Procédé selon l'une quelconque des revendications précédentes **caractérisé en ce que** l'au moins un catalyseur solide est un catalyseur conducteur électrique ou semi-conducteur.

5. Procédé selon l'une quelconque des revendications précédentes **caractérisé en ce que** l'au moins un catalyseur solide est choisi parmi un catalyseur d'hydrogénation ou un catalyseur comprenant des matériaux carbonés, ou un mélange de ceux-ci.

6. Procédé selon l'une quelconque des revendications précédentes **caractérisé en ce que** ledit catalyseur métallique comprend au moins un métal choisi parmi le fer, le ruthénium, le cobalt, le nickel, le chrome, le platine, le palladium, le rhodium, ou un mélange de ceux-ci ou de leurs oxydes.

7. Procédé selon la revendication 6 **caractérisé en ce que** ledit catalyseur métallique comprend le fer, le cobalt, le nickel et le ruthénium.

8. Procédé selon la revendication 7 **caractérisé en ce que** ledit catalyseur métallique comprend du fer.

9. Procédé selon l'une quelconque des revendications précédentes **caractérisé en ce que** le catalyseur solide se présente sous forme d'une grille, d'une plaque ou d'une structure tridimensionnelle ou poreuse.

10. Procédé selon l'une quelconque des revendications 4 à 9 **caractérisé en ce que** ledit catalyseur est une membrane métallique.

11. Procédé selon l'une quelconque des revendications précédentes **caractérisé en ce que** le catalyseur solide sert de support à l'au moins une source de microorganismes.

12. Procédé selon l'une quelconque des revendications précédentes **caractérisé en ce que** la source de microorganismes est choisie dans le groupe comprenant des cultures pures, des co-cultures, des inocula environnementaux et des consortia microbiens complexes.

13. Procédé selon l'une quelconque des revendications précédentes **caractérisé en ce que** les molécules organiques produites sont choisies dans le groupe comprenant les hydrocarbures, les alcools, les acides carboxyliques, les polyhydroxyalcanoates.

14. Dispositif apte à mettre en oeuvre le procédé selon l'une quelconque des revendications précédentes, ledit dispositif comprenant :
- des moyens d'arrivée d'au moins un courant gazeux comprenant du dihydrogène,
- au moins une source de carbone,
- au moins une source de microorganismes,
- au moins un moyen de mise en contact dudit au moins un courant gazeux et desdits microorganismes, et
- un catalyseur solide, ledit catalyseur solide étant un catalyseur métallique massif ou supporté.

## Patentansprüche

1. Verfahren zur Synthese von organischen Molekülen, umfassend das In-Kontakt-Bringen wenigstens einer Quelle für anaerobe Mikroorganismen, die die Reduktion von kohlenstoffhaltigen Molekülen durch Wasserstoff katalysieren können, mit wenigstens einem Wasserstoffstrom in einem wässrigen flüssigen Medium unter anaeroben Bedingungen, in Gegenwart wenigstens einer Kohlenstoffquelle und wenigstens eines festen Katalysators, **dadurch gekennzeichnet, dass** der feste Katalysator ein massiver oder geträgerter Metallkatalysator ist.

2. Verfahren gemäß Anspruch 1, **dadurch gekennzeichnet, dass** der gesamte oder ein Teil des Wasserstoffstroms aus einer mikrobiellen Elektrolyse, die die Oxidation von organischen Abfällen umfasst, und/oder aus einer anaeroben Vergärung von organischen Abfällen und/oder aus der Elektrolyse des Wassers stammt.

3. Verfahren gemäß Anspruch 1 oder 2, **dadurch gekennzeichnet, dass** die wenigstens eine Kohlenstoffquelle
a. entweder in Gasform vorliegt und aus Kohlenmonoxid und Kohlendioxid ausgewählt ist;
b. oder in Lösung vorliegt und aus Lösungen, die Carbonat, C₁-C₈-Carboxylate, Zucker, Lactat, einzeln oder im Gemisch, umfassen, ausgewählt ist.

4. Verfahren gemäß einem der vorstehenden Ansprüche, **dadurch gekennzeichnet, dass** der wenigstens eine feste Katalysator ein elektrisch leitfähiger oder halbleitender Katalysator ist.

5. Verfahren gemäß einem der vorstehenden Ansprüche, **dadurch gekennzeichnet, dass** der wenigstens eine feste Katalysator aus einem Hydrierungskatalysator oder einem Katalysator, der kohlenstoffhaltige Stoffe umfasst, oder einem Gemisch derselben ausgewählt ist.

6. Verfahren gemäß einem der vorstehenden Ansprüche, **dadurch gekennzeichnet, dass** der Metallkatalysator wenigstens ein Metall umfasst, das aus Eisen, Ruthenium, Cobalt, Nickel, Chrom, Platin, Palladium, Rhodium oder einem Gemisch derselben oder ihrer Oxide ausgewählt ist.

7. Verfahren gemäß Anspruch 6, **dadurch gekennzeichnet, dass** der Metallkatalysator Eisen, Cobalt, Nickel und Ruthenium umfasst.

8. Verfahren gemäß Anspruch 7, **dadurch gekennzeichnet, dass** der Metallkatalysator Eisen umfasst.

9. Verfahren gemäß einem der vorstehenden Ansprüche, **dadurch gekennzeichnet, dass** der feste Katalysator in Form eines Gitters, einer Platte oder einer dreidimensionalen oder porösen Struktur vorliegt.

10. Verfahren gemäß einem der Ansprüche 4 bis 9, **dadurch gekennzeichnet, dass** der Katalysator eine Metallmembran ist.

11. Verfahren gemäß einem der vorstehenden Ansprüche, **dadurch gekennzeichnet, dass** der feste Katalysator als Träger für die wenigstens eine Quelle für Mikroorganismen dient.

12. Verfahren gemäß einem der vorstehenden Ansprüche, **dadurch gekennzeichnet, dass** die Quelle für Mikroorganismen aus der Gruppe ausgewählt ist, die aus Reinkulturen, Cokulturen, Umweltinokula und komplexen mikrobiellen Konsortien besteht.

13. Verfahren gemäß einem der vorstehenden Ansprüche, **dadurch gekennzeichnet, dass** die erzeugten organischen Moleküle aus der Gruppe ausgewählt sind, die aus Kohlenwasserstoffen, Alkoholen, Carbonsäuren und Polyhydroxyalkanoaten besteht.

14. Vorrichtung, die zur Durchführung des Verfahrens gemäß einem der vorstehenden Ansprüche geeignet ist, wobei die Vorrichtung umfasst:
- Mittel zum Einlass wenigstens eines Gasstroms, der Wasserstoff umfasst;
- wenigstens eine Kohlenstoffquelle;
- wenigstens eine Quelle für Mikroorganismen;
- wenigstens ein Mittel zum In-Kontakt-Bringen des wenigstens einen Gasstroms mit den Mikroorganismen; und
- einen festen Katalysator, wobei der feste Katalysator ein massiver oder geträgerter Metallkatalysator ist.

## Claims

1. A process for synthesising organic molecules comprising contacting, in an aqueous liquid medium under anaerobic conditions, at least one source of anaerobic microorganisms, capable of catalysing the reduction of carbon molecules by dihydrogen, with at least one dihydrogen stream, in the presence of at least one carbon source and at least one solid catalyst **characterised in that** the solid catalyst is a bulk or supported metal catalyst.

2. The process according to claim 1, **characterised in that** all or part of the dihydrogen stream comes from a microbial electrolysis comprising the oxidation of organic waste, and/or an anaerobic digestion of organic waste, and/or water electrolysis.

3. The process according to claim 1 or claim 2, **characterised in that** the at least one carbon source is
a. either in gas form and chosen from carbon monoxide and carbon dioxide,
b. or in solution and chosen from solutions comprising carbonate, C₁-C₈ carboxylates, sugars, lactate, alone or in mixture.

4. The process according to any of the preceding claims, **characterised in that** the at least one solid catalyst is an electrically conducting or semi-conductor catalyst.

5. The process according to any of the preceding claims, **characterised in that** the at least one solid catalyst is chosen from a hydrogenation catalyst or a catalyst comprising carbon materials, or a mixture thereof.

6. The process according to any of the preceding claims, **characterised in that** said metal catalyst comprises at least one metal chosen from iron, ruthenium, cobalt, nickel, chromium, platinum, palladium, rhodium, or a mixture of the same or of their oxides.

7. The process according to claim 6, **characterised in that** said metal catalyst comprises iron, cobalt, nickel and ruthenium,

8. The process according to claim 7 **characterised in that** said catalyst comprises iron.

9. The process according to any of the preceding claims, **characterised in that** the solid catalyst is in the form of a grid, a plate or a three-dimensional or porous structure.

10. The process according to any of claims 4 to 9, **characterised in that** said catalyst is a metal membrane.

11. The process according to any of the preceding claims, **characterised in that** the solid catalyst is used as a support for the at least one source of microorganisms.

12. The process according to any of the preceding claims, **characterised in that** the source of microorganisms is chosen from the group comprising pure cultures, co-cultures, environmental inocula and complex microbial consortia.

13. The process according to any of the preceding claims, **characterised in that** the organic molecules produced are chosen from the group comprising hydrocarbons, alcohols, carboxylic acids, polyhydroxyalkanoates.

14. A device able to implement the process according to any of the preceding claims, said device comprising:
- means for feeding at least one gas stream comprising dihydrogen,
- at least one carbon source,
- at least one source of microorganisms,
- at least one means for contacting said at least one gas stream and said microorganisms, and
- a solid catalyst, said catalyst being a bulk or supported metal catalyst.
